(19) Europäisches Patentamt
European Patent Office
Office européen des brevets

(11) Veröffentlichungsnummer: **0 301 349 A1**

(12) **EUROPÄISCHE PATENTANMELDUNG**

(21) Anmeldenummer: **88111505.9**

(22) Anmeldetag: **18.07.88**

(51) Int. Cl.4: **C07D 249/08 , C07D 303/36 , A01N 43/653**

(30) Priorität: **31.07.87 DE 3725396**

(43) Veröffentlichungstag der Anmeldung: **01.02.89 Patentblatt 89/05**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Krämer, Wolfgang, Dr.**
**Rosenkranz 25**
**D-5093 Burscheid 2(DE)**
Erfinder: **Büchel, Karl Heinz, Prof. Dr.**
**Dabringhauser Strasse 42**
**D-5093 Burscheid(DE)**
Erfinder: **Holmwood, Graham, Dr.**
**Krutscheider Weg 105**
**D-5600 Wuppertal 11(DE)**
Erfinder: **Dutzmann, Stefan, Dr.**
**Leinenweberweg 33**
**D-4000 Düsseldorf 13(DE)**
Erfinder: **Reinecke, Paul, Dr.**
**Steinstrasse 8**
**D-5090 Leverkusen 3(DE)**

(54) **Hydroxyethyl-azolyl-oximether.**

(57) Neue Hydroxyethyl-azolyl-oximether der Formel

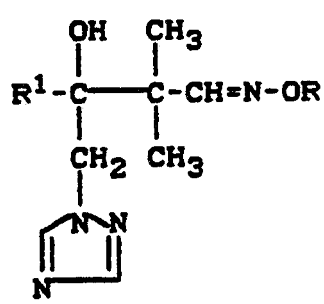

(I)

in welcher
R für Wasserstoff, Alkyl mit 1 bis 6 Kohlenstoffatomen oder Alkenyl mit 2 bis 6 Kohlenstoffatomen steht und
$R^1$ für die Reste der Formeln

EP 0 301 349 A1

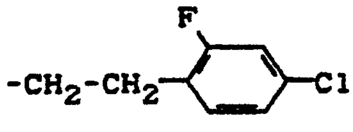 , 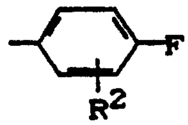 oder

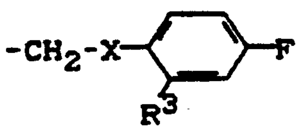

steht, worin

$R^2$ für Wasserstoff, Fluor oder Chlor steht,

$R^3$ für Wasserstoff, Fluor oder Chlor steht und

X für Sauerstoff, Schwefel oder eine $CH_2$-Gruppe steht,

sowie deren Säureadditions-Salze und Metallsalz-Komplexe,

mehrere zur Herstellung der neuen Stoffe und deren Verwendung als Fungizide.

Neue Oxirane der Formel

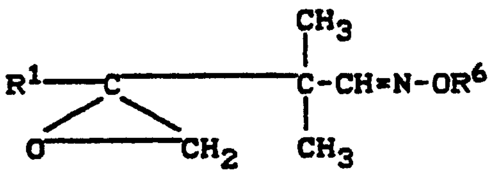 (V)

in welcher

$R^1$ die oben angegebene Bedeutung hat und

$R^6$ für Alkyl mit 1 bis 6 Kohlenstoffatomen oder Alkenyl mit 2 bis 6 Kohlenstoffatomen steht,

ein Verfahren zu deren Herstellung und deren Verwendung als Zwischenprodukte zur Synthese von Stoffen der Formel (I).

## Hydroxyethyl-azolyl-oximether

Die vorliegende Erfindung betrifft neue Hydroxyethylazolyl-oximether, mehrere Verfahren zu deren Herstellung und deren Verwendung als Fungizide.

Es ist bereits bekannt geworden, daß bestimmte Hydroxyethyl-azolyl-Derivate fungizide Eigenschaften besitzen (vgl. EP-OS 0 141 204). So lassen sich z.B. 2-(4-Chlorphenyl)-3-methyl-3-propoximinomethyl-1-(1,2,4-triazol-1-yl)-butan-2-ol, 2-(4-Chlorphenyl)-3-methyl-3-cyclohexylmethoximinomethyl-1-(1,2,4-triazol-1-yl)-butan-2-ol und 2-(4-Chlorphenyl)-3-methyl-3-n-butoximinomethyl-1-(1,2,4-triazol-1-yl)-butan-2-ol zur Bekämpfung von phytopathogenen Pilzen verwenden. Die Wirksamkeit dieser Verbindungen ist gut, läßt aber beim Einsatz in geringen Aufwandmengen in manchen Fällen zu wünschen übrig.

Es wurden nun neue Hydroxyethyl-azolyl-oximether der Formel

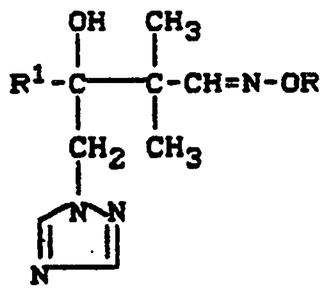

(I)

in welcher

R für Wasserstoff, Alkyl mit 1 bis 6 Kohlenstoffatomen oder Alkenyl mit 2 bis 6 Kohlenstoffatomen steht und

$R^1$ für die Reste der Formel

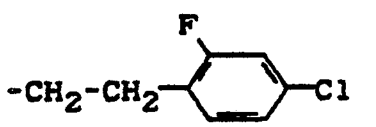

,

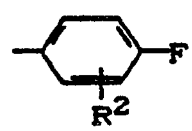

oder

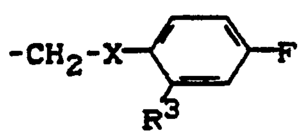

steht, worin

$R^2$ für Wasserstoff, Fluor oder Chlor steht,

$R^3$ für Wasserstoff, Fluor oder Chlor steht und

X für Sauerstoff, Schwefel oder eine $CH_2$-Gruppe steht,

sowie deren Säureadditions-Salze und Metallsalz-Komplexe gefunden.

Weiterhin wurde gefunden, daß man Hydroxyethyl-azolyloximether der Formel (I) sowie deren Säureadditions-Salze und Metallsalzkomplexe erhält, wenn man

a) Hydroxyethyl-azolyl-acetale der Formel

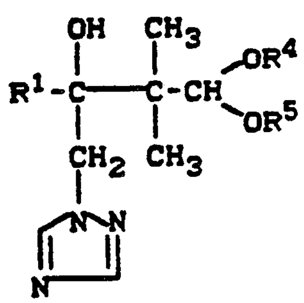

(IIa)

in welcher
$R^1$ die oben angegebene Bedeutung hat,
$R^4$ für Alkyl mit 1 bis 4 Kohlenstoffatomen steht und
$R^5$ für Alkyl mit 1 bis 4 Kohlenstoffatomen steht oder
$R^4$ und $R^5$ gemeinsam für eine $-CH_2-CH_2-$Gruppe stehen,
oder Hydroxyethyl-azolyl-aldehyde der Formel

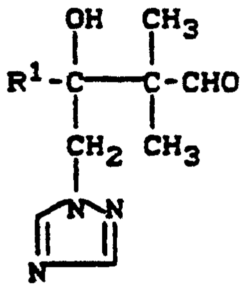

(IIb)

in welcher
$R^1$ die oben angegebene Bedeutung hat,
mit Hydroxylamin-Verbindungen der Formel

$H_2N-OR$ (III)

in welcher
R die oben angegebene Bedeutung hat,
oder mit Hydrochloriden von Hydroxylamin-Verbindungen der Formel (III) in Gegenwart eines Verdünnungsmittels und gegebenenfalls in Gegenwart eines Katalysators umsetzt,
oder

b) Hydroxyethyl-azolyl-oxim-Derivate der Formel

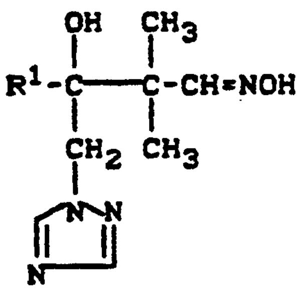

(Ia)

in welcher
$R^1$ die oben angegebene Bedeutung hat,
mit Halogen-Verbindungen der Formel

$Hal-R^6$ (IV)

in welcher
Hal für Chlor, Brom oder Iod steht und
$R^6$ für Alkyl mit 1 bis 6 Kohlenstoffatomen oder Alkenyl mit 2 bis 6 Kohlenstoffatomen steht,
in Gegenwart eines Verdünnungsmittels und gegebenenfalls in Gegenwart eines Säurebindemittels umsetzt,
oder

c) Oxirane der Formel

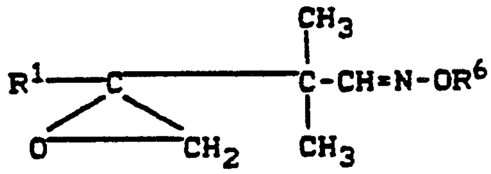 (V)

in welcher
$R^6$ und $R^1$ die oben angegebene Bedeutung haben,
mit 1,2,4-Triazol der Formel

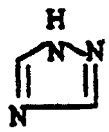 (VI)

in Gegenwart eines Verdünnungsmittels und gegebenenfalls in Gegenwart eines Säurebindemittels umsetzt, und gegebenenfalls anschließend an die so erhaltenen Verbindungen der Formel (I) eine Säure oder ein Metallsalz addiert.

Schließlich wurde gefunden, daß die neuen Hydroxyethylazolyl-oximether der Formel (I) sowie deren Säureadditions-Salze und Metallsalzkomplexe starke fungizide Eigenschaften aufweisen.

Überraschenderweise zeigen die erfindungsgemäßen Stoffe eine bessere fungizide Wirksamkeit als 2-(4-Chlorphenyl)-3-methyl-3-propoximinomethyl-1-(1,2,4-triazol-1-yl)-butan-2-ol, 2-(4-Chlorphenyl)-3-methyl-3-cyclohexylmethoximinomethyl-1-(1,2,4-triazol-1-yl)-butan-2-ol und 2-(4-Chlorphenyl)-3-methyl-3-n-butoximino methyl-1-(1,2,4-triazol-1-yl)-butan-2-ol, welches die konstitutionell ähnlichsten vorbekannten Wirkstoffe gleicher Wirkungsrichtung sind.

Die erfindungsgemäßen Hydroxyethyl-azolyl-oximether sind durch die Formel (I) allgemein definiert.
Bevorzugt sind diejenigen Verbindungen in denen
R für Wasserstoff, Methyl, Ethyl, n-Propyl, iso-Propyl, n-Butyl, iso-Butyl, sek.-Butyl, tert.-Butyl oder Allyl steht und
$R^1$ für die Reste der Formel

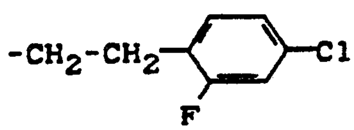 , 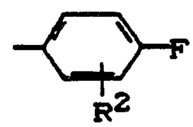 oder

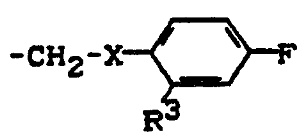

steht, worin
$R^2$ für Wasserstoff, Fluor oder Chlor steht,
$R^3$ für Wasserstoff, Fluor oder Chlor steht
und
X für Sauerstoff, Schwefel oder eine $CH_2$-Gruppe steht.

Bevorzugte erfindungsgemäße Verbindungen sind auch Additionsprodukte aus Säuren und denjenigen Hydroxyethyl-azolyl-oximethern der Formel (I), in denen R und $R^1$ die Bedeutungen haben, die für diese Reste bereits als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure, sowie Saccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Hydroxyethyl-azolyl-oximethern der Formel (I), in denen R und R' die Bedeutungen haben, die für diese Reste bereits als bevorzugt genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen.

Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man 2-(2-Dioxolanyl)-3-(4-fluorphenyl)-2-methyl-4-(1,2,4-triazol-1-yl)-butan-3-ol und O-Methylhydroxylaminhydrochlorid als Ausgangsstoffe und konzentrierte Salzsäure als Katalysator, so kann der Verlauf des erfindungsgemäßen Verfahrens (a) durch das folgende Formelschema veranschaulicht werden:

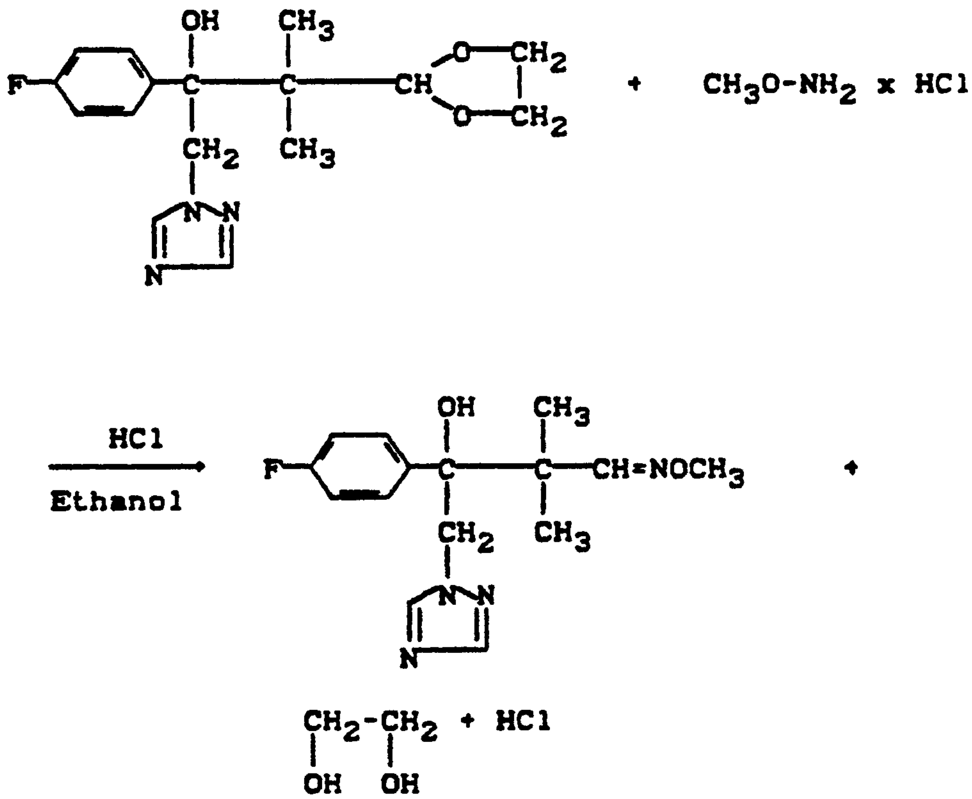


Verwendet man 3-(2,4-Difluor-phenyl)-2,2-dimethyl-3-hydroxy-4-(1,2,4-triazol-1-yl)-butan-1-al und O-Methylhydroxylaminhydrochorid als Ausgangsstoffe, so kann der Verlauf des erfindungsgemäßen Verfahrens (a) durch das folgende Formelschema veranschaulicht weden:

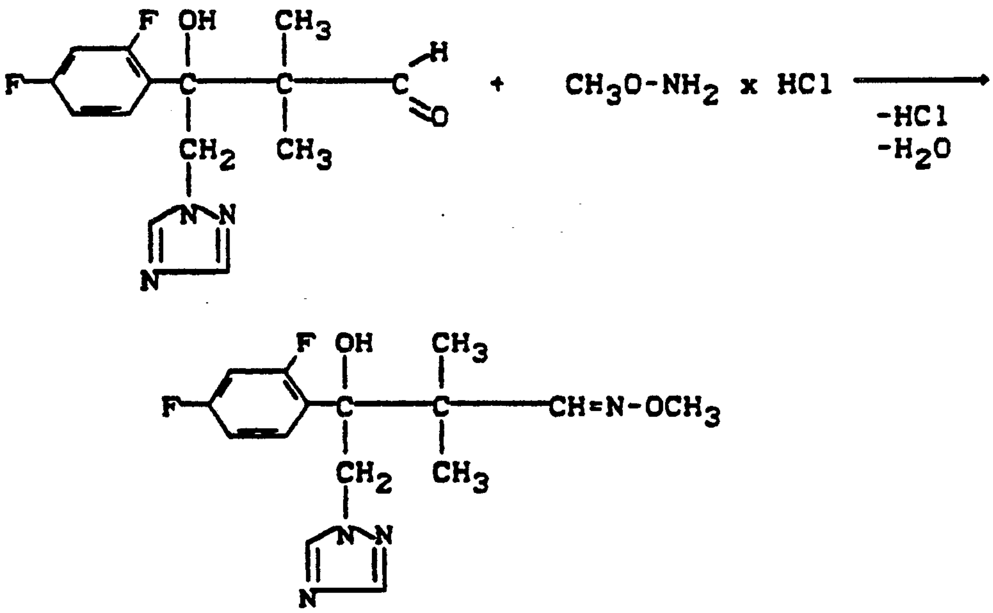


Verwendet man 3-(4-Fluor-phenyl)-2-hydrox-iminomethyl-2-methyl-4-(1,2,4-triazol-1-yl)-butan-3-ol und Allylchlorid als Ausgangsstoffe, so kann der Verlauf des erfindungsgemäßen Verfahrens (b) durch das folgende Formelschema veranschaulicht werden:

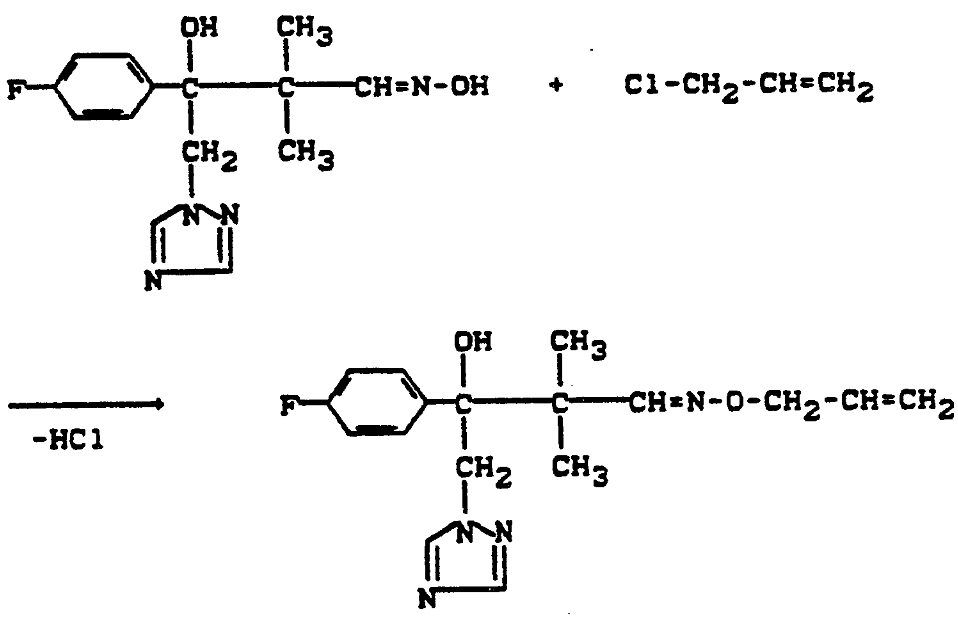


Verwendet man 2-(4-Fluor-phenyl)-2-(2-methoximinomethyl-2-propyl)-oxiran und 1,2,4-Triazol als Ausgangsstoffe, so kann der Verlauf des erfindungsgemäßen Verfahrens (c) durch das folgende Formelschema veranschaulicht werden:

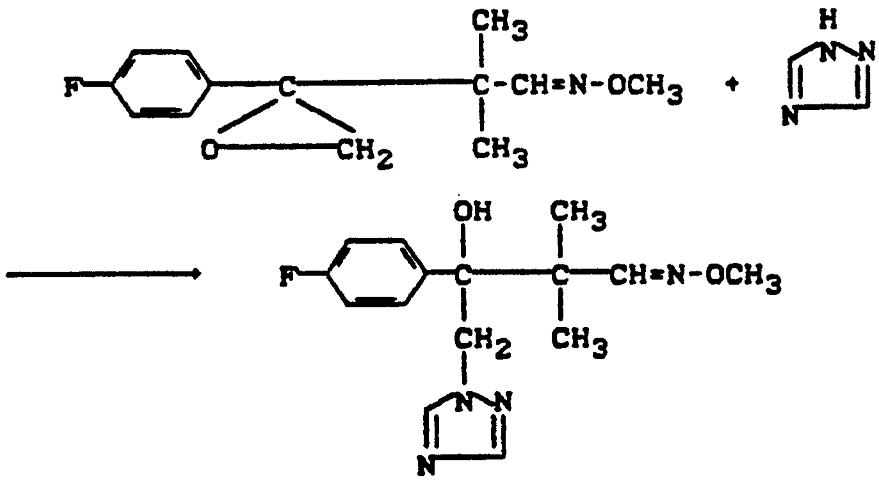

Die bei der Durchführung des erfindungsgemäßen Verfahrens (a) als Ausgangsstoffe benötigten Hydroxyethylazolyl-acetale sind durch die Formel (IIa) allgemein definiert. In dieser Formel hat $R^1$ vorzugsweise diejenigen Bedeutungen, die bereits im Zusammenhang mit der Beschreibung der erfindungsgemäßen Stoffe für $R^1$ als bevorzugt genannt wurden. $R^4$ und $R^5$ stehen vorzugsweise für Methyl oder Ethyl oder gemeinsam für eine $-CH_2-CH_2-$Gruppe.

Die Hydroxyethyl-azolyl-acetale der Formel (IIa) lassen sich herstellen, indem man Oxirane der Formel

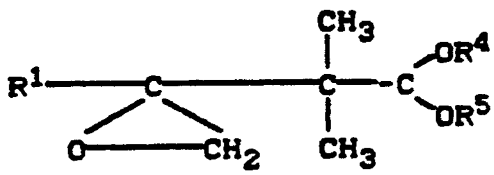

(VII)

in welcher
$R^1$. $R^4$ und $R^5$ die oben angegebene Bedeutung haben,
mit 1,2,4-Triazol der Formel

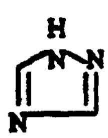

(VI)

in Gegenwart eines inerten organischen Verdünnungsmittels, wie z.B. eines Alkohols, und gegebenenfalls in Gegenwart eines Säurebindemittels, wie Natriumalkoholat oder Kaliumhydroxid, bei Temperaturen zwischen 60 °C und 150 °C umsetzt (vgl. Herstellungsbeispiele).

Die Oxirane der Formel (VI) lassen sich herstellen, indem man Ketone der Formel

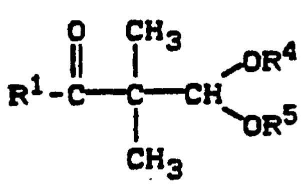

(VIII)

in welcher
$R^1$, $R^4$ und $R^5$ die oben angegebene Bedeutung haben,
entweder

α) mit Dimethyloxosulfonium-methylid der Formel

$$\overset{\delta+}{(CH_3)_2S}\overset{\delta-}{OCH_2} \qquad (IX)$$

in an sich bekannter Weise in Gegenwart eines Verdünnungsmittels, wie beispielsweise Dimethylsulfoxid, bei Temperaturen zwischen 20 °C und 80 °C umsetzt (vgl. hierzu die Angaben in J. Am. Chem. Soc. 87, 1363-1364 (1965)), oder

β) mit Trimethylsulfonium-methylsulfat der Formel

$$[(CH_3)_3S^{\oplus}]\ CH_3SO_4^{\ominus} \qquad (X)$$

in an sich bekannter Weise in Gegenwart eines inerten organischen Lösungsmittels, wie z.B. Acetonitril, und in Gegenwart einer Base, wie z.B. Natriumethylat, bei Temperaturen zwischen 0 °C und 60 °C, vorzugsweise bei Raumtemperatur, umsetzt (vgl. auch die Angaben in Heterocycles 8, 397 (1977)).

Die so erhaltenen Oxirane der Formel (VII) können gegebenenfalls ohne Isolierung direkt weiter umgesetzt werden.

Die Ketone der Formel (VIII) lassen sich herstellen, indem man 1-(N-Morpholino)-isobuten der Formel

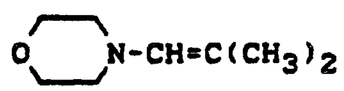 (XI)

mit Chloriden der Formel

$$R^1\text{-CO-Cl} \qquad (XII)$$

in welcher

$R^1$ die oben angegebene Bedeutung hat,

in Gegenwart eines Lösungsmittels, wie beispielsweise Diethylether, bei Temperaturen zwischen 20 °C und 120 °C umsetzt und die so erhaltenen Keto-Derivate der Formel

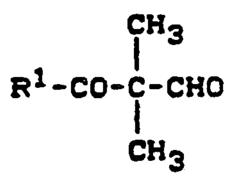 (XIII)

in welcher

$R^1$ die oben angegebene Bedeutung hat,

mit Hilfe von Alkoholen, wie Methanol, Ethanol oder Ethylenglykol, in Gegenwart eines inerten organischen Verdünnungsmittels, wie Toluol, und in Gegenwart einer starken Säure, wie p-Toluolsulfonsäure, als Katalysator bei Temperaturen zwischen 80 °C und 110 °C in die entsprechenden Acetale überführt.

Die bei dem erfindungsgemäßen Verfahren (a) weiterhin als Ausgangsstoffe benötigten Hydroxyethyl-azolyl-aldehyde sind durch die Formel (IIb) allgemein definiert. In dieser Formel hat $R^1$ vorzugsweise diejenigen Bedeutungen, die bereits im Zusammenhang mit der Beschreibung der erfindungsgemäßen Stoffe der Formel (I) für diesen Rest als bevorzugt genannt wurden.

Die Hydroxyethyl-azolyl-aldehyde der Formel (IIb) lassen sich herstellen, indem man Hydroxyethyl-azolyl-acetale der Formel

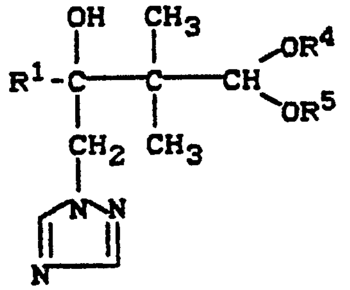

(IIa)

in welcher
$R^1$, $R^4$ und $R^5$ die oben angegebene Bedeutung haben,
in Gegenwart eines Gemisches aus Wasser und einem mit Wasser mischbaren, inerten organischen Lösungsmittel, wie Methanol oder Ethanol, sowie in Gegenwart einer anorganischen oder organischen Säure, wie Salzsäure, Schwefelsäure, p-Toluolsulfonsäure oder Essigsäure, bei Temperaturen zwischen 30 °C und 120 °C, vorzugsweise bei der Siedetemperatur des verwendeten Lösungsmittels, umsetzt.

Die bei der Durchführung des erfindungsgemäßen Verfahrens (a) als Reaktionskomponenten benötigten Hydroxylamin-Verbindungen sind durch die Formel (III) allgemein definiert. In dieser Formel steht R vorzugsweise für diejenigen Reste, die bereits im Zusammenhang mit der Beschreibung der erfindungsgemäßen Stoffe der Formel (I) als bevorzugt für diesen Rest genannt wurden.

Die Hydroxylamin-Verbindungen der Formel (III) und deren Hydrochloride sind allgemein bekannte Verbindungen der organischen Chemie.

Als Verdünnungsmittel können bei der Durchführung des erfindungsgemäßen Verfahrens (a) alle für derartige Umsetzungen üblichen Solventien verwendet werden. Vorzugsweise kommen Alkohole und Wasser sowie deren Gemische in Betracht.

Als Katalysatoren kommen bei der Durchführung des erfindungsgemäßen Verfahrens (a) alle für derartige Umsetzungen üblichen Reaktionsbeschleuniger in Frage. Vorzugsweise verwendbar sind starke anorganische und organische Säuren, wie Salzsäure und p-Toluolsulfonsäure.

Die Reaktionstemperaturen können bei der Durchführung des erfindungsgemäßen Verfahrens (a) innerhalb eines größeren Bereiches variiert werden. Im allgemeinen arbeitet man bei Temperaturen zwischen 20 °C und 120 °C, vorzugsweise zwischen 50 °C und 100 °C.

Bei der Durchführung des erfindungsgemäßen Verfahrens (a) setzt man auf 1 Mol an einer Verbindung der Formel (IIa) oder (IIb) vorzugsweise 1 bis 1,5 Mol an Hydroxylamin-Verbindung der Formel (III) sowie gegebenenfalls eine katalytische Menge an Reaktionsbeschleuniger ein. Die Aufarbeitung und die Isolierung der Verbindungen der Formel (I) erfolgt nach üblichen Methoden. In einer bevorzugten Ausführungsform des erfindungsgemäßen Verfahrens (a) werden die Hydroxylamin-Verbindungen der Formel (III) in Form ihrer Hydrochloride eingesetzt.

Die bei der Durchführung des erfindungsgemäßen Verfahrens (b) als Ausgangsstoffe zu verwendeten Hydroxyethyl-azolyloximether der Formel (Ia) sind erfindungsgemäße Verbindungen.

Die außerdem für die Durchführung des erfindungsgemäßen Verfahrens (b) als Ausgangsstoffe zu verwendeten Halogenverbindungen sind durch die Formel (IV) allgemein definiert. In dieser Formel steht Hal vorzugsweise für Methyl, Ethyl, n-Propyl, iso-Propyl, n-Butyl, iso-Butyl, sek.-Butyl, tert.-Butyl oder Allyl.

Die Halogen-Verbindungen der Formel (IV) sind allgemein bekannte Verbindungen der organischen Chemie.

Bei der Durchführung des erfindungsgemäßen Verfahrens (b) kommen als Verdünnungsmittel inerte organische Solventien in Frage. Vorzugsweise verwendbar sind Ether, wie Tetrahydrofuran und Dioxan; aromatische Kohlenwaserstoffe, wie Toluol und Benzol; in einzelnen Fällen auch chlorierte Kohlenwasserstoffe, wie Chloroform, Methylenchlorid oder Tetrachlorkohlenstoff; sowie Hexamethylphosphorsäure-triamid, Säureamide, wie Dimethylformamid, und Sulfoxide, wie Dimethylsulfoxid.

Als Säurebindemittel kommen bei der Durchführung des erfindungsgemäßen Verfahrens (b) starke Basen in Betracht. Vorzugsweise verwendbar sind Alkalimetallamide, -hydride, -hydroxide und -carbonate, wie beispielsweise Natriumamid, -carbonat, -hydroxid oder -hydrid und Kaliumamid, -carbonat, -hydroxid oder -hydrid, sowie quarternäre Ammoniumhydroxide und Phosphoniumhydroxide, wie beispielsweise Tetramethylammoniumhydroxid, Benzyl-trimethyl-ammoniumhydroxid oder Dibenzyl-dimethyl-ammoniumhydroxid und Tetraphenylphosphoniumhydroxid oder Methyltriphenyl-phosphoniumhydroxid.

Die Reaktionstemperaturen können beim Verfahren (b) in einem größeren Bereich variiert werden. Im allgemeinen arbeitet man zwischen 20 °C und 150 °C, vorzugsweise bei Raumtemperatur. In einzelnen Fällen ist es vorteilhaft, bei der Siedetemperatur des Lösungsmittels, beispielsweise zwischen 60 °C und

100 °C, zu arbeiten.

Bei der Durchführung des erfindungsgemäßen Verfahrens (b) setzt man auf 1 Mol der Verbindungen der Formel (Ia) vorzugsweise 1 bis 3 Mol Halogen-Verbindung der Formel (IV) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform des Verfahrens (b) wird die erfindungsgmeäße Umsetzung in einem Zweiphasensystem, wie beispielsweise wäßrige Natron- oder Kalilauge/Toluol oder Methylenchlorid, unter Zusatz von 0,01 bis 1 Mol eines Phasen-Transfer-Katalysators, wie beispielsweise Ammonium- oder Phosphoniumverbindungen, durchgeführt, wobei in der organischen Phase oder an den Grenzflächen die Alkoholate entstehen und mit den in der organischen Phase befindlichen Halogen-Verbindungen umgesetzt werden.

Die bei der Durchführung des erfindungsgemäßen Verfahrens (c) als Ausgangsverbindungen benötigten Oxirane sind durch die Formel (V) allgemein definiert. In dieser Formel haben $R^6$ und $R^1$ vorzugsweise diejenigen Bedeutungen, die bereits im Zusammenhang mit der Beschreibung der erfindungsgemäßen Verbindungen der Formel (I) bzw. der Halogen-Verbindungen der Formel (IV) als bevorzugt für diese Reste genannt wurden.

Die Oxirane der Formel (V) sind bisher noch nicht bekannt. Sie lassen sich herstellen, indem man Keto-oxim-Derivate der Formel

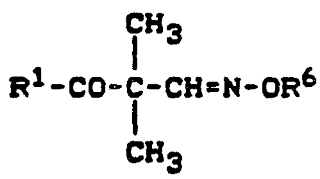
(XIV)

in welcher
$R^1$ und $R^6$ die oben angegebene Bedeutung haben,
entweder

α) mit Dimethyloxosulfonium-methylid der Formel

$$\overset{\delta\oplus}{(CH_3)_2S}\overset{\delta\ominus}{OCH_2} \qquad (IX)$$

in Gegenwart eines Verdünnungsmittels, wie Dimethylsulfoxid, bei Temperaturen zwischen 20 °C und 80 °C umsetzt,
oder

β) mit Trimethylsulfonium-methylsulfat der Formel

$[(CH_3)_3S^{\oplus}\ CH_3SO_4^{\ominus}$ (X)

in Gegenwart eines inerten organischen Lösungsmittels, wie Acetonitril, und in Gegenwart einer Base, wie Natrium-methylat, bei Temperaturen zwischen 0 °C und 60 °C, vorzugsweise bei Raumtemperatur, umsetzt.

Die Keto-oxim-Derivate der Formel (XIV) lassen sich herstellen, indem man 1-(N-Morpholino)-isobuten der Formel (XI) mit Chloriden der Formel (XII) in Gegenwart eines Lösungsmittels, wie beispielsweise Diethylether, bei Temperaturen zwischen 20 °C und 120 °C umgesetzt und die so erhaltenen Keto-Derivate der Formel (XIII) in üblicher Weise an der Aldehydgruppe mittels Hydroxylamin-Verbindungen der Formel (III) oder deren Hydrochloriden, wie zum Beispiel Methoxyhydroxylamin-hydrochlorid, in Gegenwart eines inerten organischen Lösungsmittels, wie beispielsweise Ethanol, und in Gegenwart von Natriumacetat bei Temperaturen zwischen 80 °C und 110 °C derivatisiert. In manchen Fällen erweist es sich als vorteilhaft, den Rest $R^1$ bzw. Teile davon erst nach der Derivatisierung der Aldehydgruppe einzuführen.

Das bei der Durchführung des erfindungsgemäßen Verfahrens (c) als Reaktionskomponente benötigte 1,2,4- Triazol der Formel (VI) ist bekannt. Es kann als freie Base oder auch in Form seines Natrium- oder Kalium-Salzes eingesetzt werden.

Als Verdünnungsmittel kommen bei der Durchführung des erfindungsgemäßen Verfahrens (c) alle unter den Reaktionsbedingungen inerten organischen Solventien in Frage. Vorzugsweise verwendbar sind Alkohole, wie z.B. Ethanol, Methoxyethanol oder Propanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Säurebindemittel kommen bei der Durchführung des erfindungsgemäßen Verfahrens (c) alle üblichen anorganischen und organischen Basen in Betracht. Vorzugsweise verwendbar sind Alkalicarbonate, wie z.B. Natrium-und Kaliumcarbonat; Alkalihydroxide, wie z.B. Natriumhydroxid; Alkalialkoholate, wie z.B. Natrium- und Kalium-methylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperatuern können bei der Durchführung des erfindungsgemäßen Verfahrens (c) in einem größeren Bereich variiert werden. Im allgemeinen arbeitet man bei Temperaturen zwischen 0°C und 200°C, vorzugsweise zwischen 60°C und 150°C.

Bei der Durchführung des erfindungsgemäßen Verfahrens (c) setzt man vorzugsweise auf 1 Mol Oxiran der Formel (V) 1 bis 2 Mol 1,2,4-Triazol und gegebenenfalls 1 bis 2 Mol Base ein. Die Isolierung der Endprodukte erfolgt in allgemein üblicher Weise.

Die nach den erfindungsgemäßen Verfahren erhältlichen Hydroxyethyl-azolyl-oximether der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Ventirua inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten; wie Uromyces appendiculatus;
Puccinia-Arten, wie Puccinia recondita;
Tilletia-Arten, wie Tilletia caries;

Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreide- und Reiskrankheiten.

So läßt sich Pseudocercosporella herpotrichoides an Weizen und Gerste besonders gut bekämpfen.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Träger stoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure. Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Ver fahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen wirkstoffe geht aus den nachfolgenden Beispielen hervor.

## Herstellungsbeispiele

### Beispiel 1

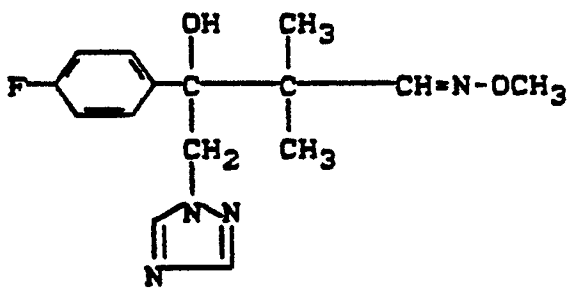

Ein Gemisch aus 215 g (0,67 Mol) 2-(2-Dioxolanyl)-3-(4-fluor-phenyl)-2-methyl-4-(1,2,4-triazol-1-yl)-butan-3-ol, 75.2 g (0,9 Mol) O-Methyl-hydroxylamin-hydrochlorid, 4 ml konzentrierter Salzsäure und 800 ml Ethanol wird 5 Stunden unter Rückfluß erhitzt. Danach wird das Lösungsmittel unter vermindertem Druck abgezogen. Der verbleibende Rückstand wird mit 1000 ml gesättigter, wäßriger Natriumhydrogencarbonat-Lösung und 1000 ml Methylenchlorid versetzt. Man trennt die Phasen, extrahiert die wäßrige Phase zweimal mit je 800 ml Methylenchlorid, trocknet die vereinigten organischen Phasen über Natriumsulfat und engt durch Abziehen des Lösungsmittels unter vermindertem Druck ein. Der Rückstand wird mit 800 ml Diisopropylether versetzt und abgesaugt. Man erhält auf diese Weise 190 g (93 % der Theorie) an 3-(4-Fluor-phenyl)-2-methoximinomethyl-2-methyl-4-(1,2,4-triazol-1-yl)-butan-3-ol in Form eines farblosen Feststoffes vom Schmelzpunkt 98-101 °C.

### Herstellung von Ausgangssubstanzen:

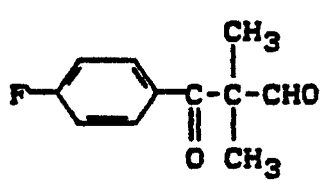

In eine Lösung von 266 g (1,89 Mol) 1-Morpholinyl-2-methyl-propen-1 in 800 ml Tetrahydrofuran werden bei 5 °C unter Rühren und unter Kühlung innerhalb von 3 Stunden 300 g (1,89 Mol) 4-Fluorbenzoylchlorid eingetropft. Das Reaktionsgemisch wird zunächst 15 Stunden bei Raumtemperatur gerührt, danach 2 Stunden unter Rückfluß erhitzt und anschließend auf 10 °C abgeküht. Der auskristallisierende Feststoff wird abgesaugt, zweimal mit je 100 ml Tetrahydrofuran gewaschen und dann in ein Gemisch aus

1000 ml Wasser und 1000 ml Methylenchlorid eingerührt. Man trennt die Phasen und engt die organische Phase durch Abziehen des Lösungsmittels unter verminderten Druck ein. Es verbleiben 295 g an 2-(4-Fluor-benzoyl)-2-methyl-propionaldehyd in Form eines gelben Öles.

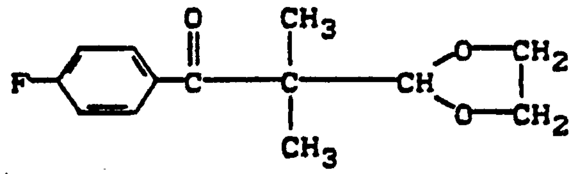

In eine Lösung aus 136 g (2,2 Mol) Ethylenglykol und 3 g p-Toluolsulfonsäure in 1500 ml Methylenchlorid werden bei Raumtemperatur 295 g (1,5 Mol) 2-(4-Fluor-benzoyl)-2-methyl-propionaldehyd gegeben. Das Reaktionsgemisch wird 24 Stunden am Wasserabscheider gekocht. Die organische Phase wird zweimal mit je 2000 ml Wasser gewaschen, dann über Natriumsulfat getrocknet und unter vermindertem Druck destilliert. Man erhält auf diese Weise 278 g (77 % der Theorie) an (4-Fluor-phenyl)-(1-dioxolanyl-1-methyl-ethyl)-keton in Form einer farblosen Flüssigkeit vom Siedepunkt 100-102 °C/0,3 mbar.

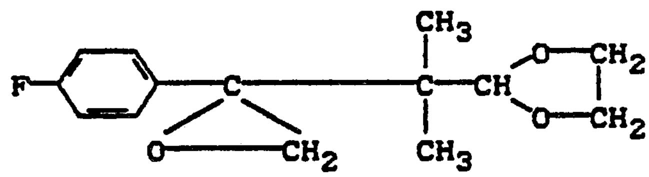

In eine Lösung von 242 g (1,1 Mol) Trimethylsulfoniumiodid in 320 ml Dimethylsulfoxid werden bei Raumtemperatur 123,1 g (1,1 Mol) Kalium-tert.-butylat gegeben. Das Gemisch wird 5 Stunden bei Raumtemperatur gerührt. Danach werden 276 g (1,16 Mol) (4-Fluor-phenyl)-(1-dioxolanyl-1-methyl-ethyl)-keton unter Rühren so zugetropft, daß die Temperatur des Reaktionsgemisches 40 °C nicht übersteigt. Man rührt anschließend noch 16 Stunden bei 40 °C und gibt dnn unter Rühren und unter Kühlung 1000 ml Wasser hinzu. Die organische Phase wird abgetrennt, zweimal mit je 2000 ml Wasser gewaschen, über Natriumsulfat getrocknet und durch Abziehen des Lösungs mittels unter vermindertem Druck eingeengt. Man erhält auf diese Weise 280 g eines hellgelben Öles, das gemäß gaschromatischer Analyse zu 84 % aus 2-(4-Fluorphenyl)-2-[1-(2-dioxolanyl)-1-methyl]-ethyloxiran besteht. Die Ausbeute errechnet sich danach zu 80,6 % der Theorie.

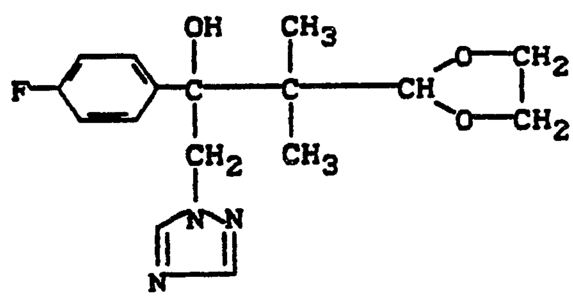

Eine Lösung von 280 g des rohen 2-(4-Fluorphenyl)-2-[1-(2-dioxolanyl)-1-methyl]-ethyl-oxirans in 1,1 Litern n-Butanol wird bei Raumtemperatur mit 75,9 g (1,1 Mol) 1,2,4-Triazol und 7 g 88 %iger wäßriger Kalilauge versetzt. Man erhitzt das Reaktionsgemisch 16 Stunden unter Rückfluß, zieht dann das Lösungsmittel unter vermindertem Druck ab, nimmt den Rückstand in 1000 ml Methylenchlorid auf und wäscht zweimal mit je 1500 ml Wasser.

Die organische Phase wird über Natriumsulfat getrocknet und durch Abziehen des Lösungsmittels unter vermindertem Druck eingeengt. Der verbleibende Rückstand wird mit 800 ml Diisopropylether versetzt. Man saugt den Feststoff ab und engt das Filtrat durch Abziehen des Lösungsmittels unter vermindertem Druck auf ein Volumen von 100 ml ein. Beim Abkühlen auf 0 °C kristallisiert ein Feststoff aus, der abgesaugt wird. Man erhält auf diese Weise 161 g an 2-(2-Dioxolanyl)-3-(4-fluor-phenyl)-2-methyl-4-(1,2,4-triazol-1-yl)-butan-3-ol in Form eines farblosen Feststoffes vom Schmelzpunkt 90 °C.

Nach der im Beispiel 1 angegebenen Methode bzw. nach den Angaben in der Beschreibung werden die

in der folgenden Tabelle aufgeführten erfindungsgemäßen Stoffe hergestellt.

**Tabelle 1**

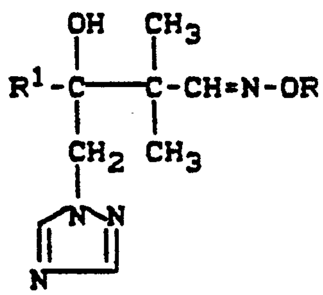

(I)

| Beispiel Nr. | $R^1$ | R | Schmelzpunkt [°C] |
|---|---|---|---|
| 2 | 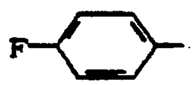 | $-C_3H_7-n$ | 105 |
| 3 | 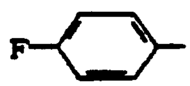 | $-C_4H_9-n$ | 114 |
| 4 | 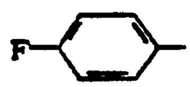 | $-C_2H_5$ | 98-100 |
| 5 | 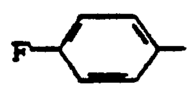 | $-CH_2-CH=CH_2$ | 97-99 |

<u>T a b e l l e 1</u> (Fortsetzung)

| Beispiel Nr. | $R^1$ | R | Schmelzpunkt [° C] |
|---|---|---|---|
| 6 | 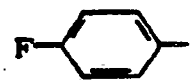 | H | 142-144 |
| 7 | 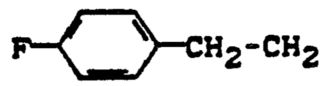 | H | 101-103 |
| 8 | 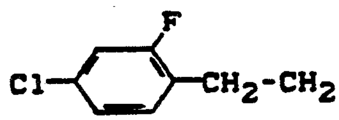 | $-CH_3$ | 83 |
| 9 | 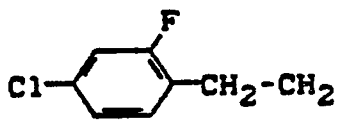 | $-C_2H_5$ | 55 |
| 10 | 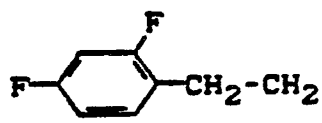 | $-CH_3$ | zähes Öl |
| 11 | 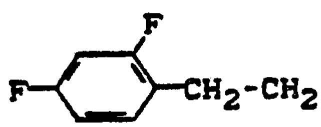 | $-C_2H_5$ | zähes Öl |
| 12 | 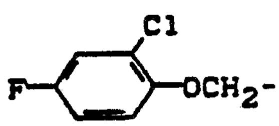 | $-CH_3$ | 111 |
| 13 | 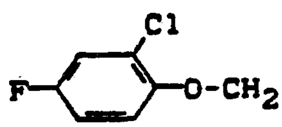 | $-C_2H_5$ | 116 |
| 14 | 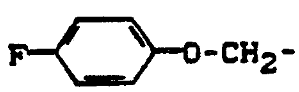 | $-CH_3$ | 123 |
| 15 | 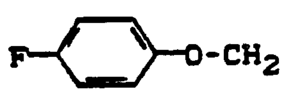 | $-C_2H_5$ | 121 |

**Tabelle 1** (Fortsetzung)

| Beispiel Nr. | $R^1$ | R | Schmelzpunkt [°C] |
|---|---|---|---|
| 16 | 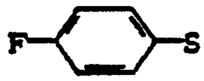 S-$CH_2$- | -$CH_3$ | zähes Öl |
| 17 | 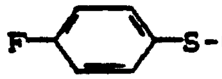 S-$CH_2$- | H | zähes Öl |
| 18 | 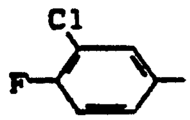 | $CH_3$ | 90 |
| 19 | 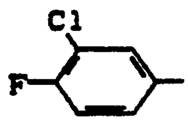 | H | 103-106 |
| 20 | 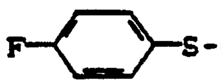 S-$CH_2$- | $C_2H_5$ | zähes Öl |
| 21 | 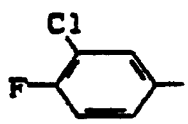 | -$C_2H_5$ | 133 |
| 22 | 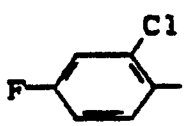 | -$CH_3$ | 131-133 |
| 23 | 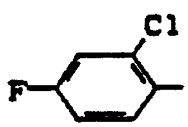 | -$C_2H_5$ | 111-115 |
| 24 | 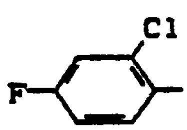 | H | 156-158 |

T a b e l l e 1 (Fortsetzung)

| Beispiel Nr. | $R^1$ | R | Schmelzpunkt [°C] |
|---|---|---|---|
| 25 | 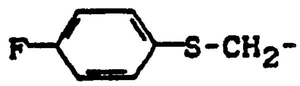 | $-C_3H_7$-n | zähes Öl |
| 26 | 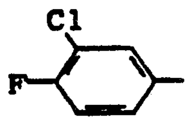 | $-C_3H_7$-n | 128-130 |
| 27 | 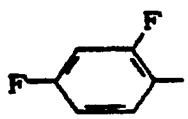 | $-CH_2-CH=CH_2$ | 112-114 |
| 28 | 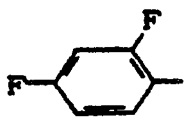 | $-C_4H_9$-n | 115-117 |
| 29 | 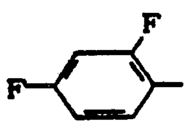 | $-CH_3$ | 127 |
| 30 | 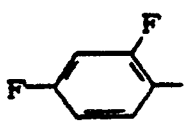 | $-C_2H_5$ | 114 |
| 31 | 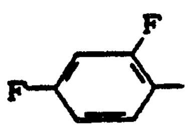 | $-C_3H_7$-n | 115-117 |

Verwendungsbeispiel

In dem folgenden Verwendungsbeispiel wurden die Verbindungen der nachstehend ausgegebenen Formeln als Vergleichssubstanzen eingesetzt:

(A) =

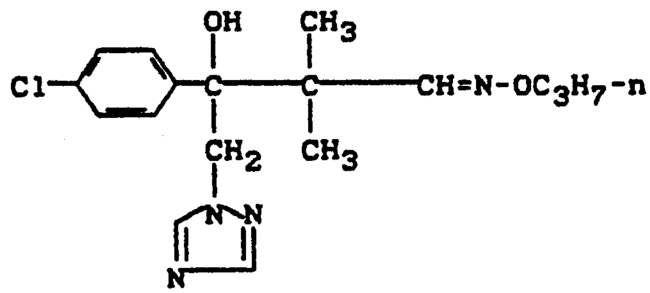

(B) =

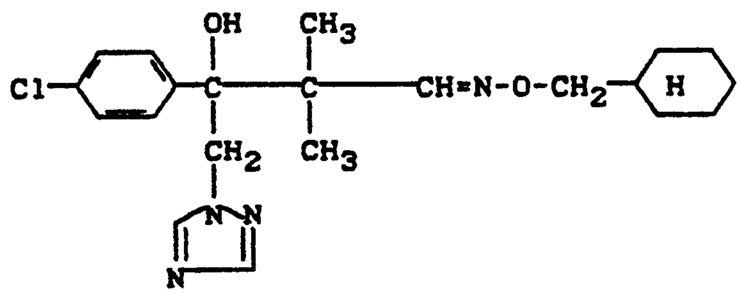

(C) =

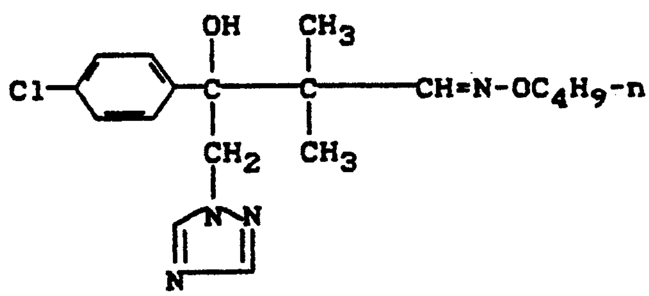

**(Bekannt aus EP-OS 0 141 204).**

Beispiel A

Pseudocercosporella herpotrichoides-Test (Weizen) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen an der Halmbasis mit Sporen von Pseudocercosporella herpotrichoides inokuliert.

Die Pflanzen werden in einem Gewächshaus bei einer Temperatur von ca. 10 °C und einer relativen Luftfeuchtigkeit von ca. 80 % aufgestellt.

21 Tage nach der Inokulation erfolgt die Auswertung.

In diesem Test zeigen die erfindungsgemäßen Stoffe (1), (2), (3), (4) und (5) eine bessere Wirksamkeit als die Vergleichssubstanzen (A), (B) und (C).

## Ansprüche

1. Hydroxyethyl-azolyl-oximether der Formel

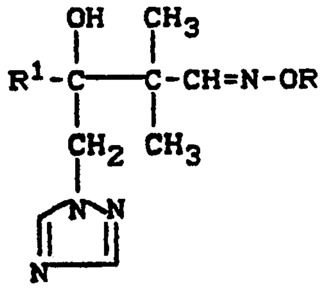

(I)

in welcher
R für Wasserstoff, Alkyl mit 1 bis 6 Kohlenstoffatomen oder Alkenyl mit 2 bis 6 Kohlenstoffatomen steht und
$R^1$ für die Reste der Formel

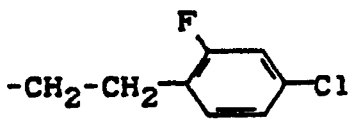

,

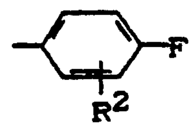

oder

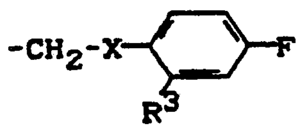

steht, worin
$R^2$ für Wasserstoff, Fluor oder Chlor steht,
$R^3$ für Wasserstoff, Fluor oder Chlor steht und
X für Sauerstoff, Schwefel oder eine $CH_2$-Gruppe steht,
sowie deren Säureadditions-Salze und Metallsalz-Komplexe.

2. Hydroxyethyl-azolyl-oximether der Formel (I) gemäß Anspruch 1, in denen
R für Wasserstoff, Methyl, Ethyl, n-Propyl, iso-Propyl, n-Butyl, iso-Butyl, sek.-Butyl, tert.-Butyl oder Allyl steht, und
$R^1$ für die Reste der Formel

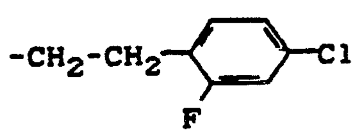

,

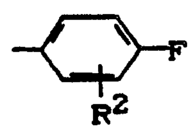

oder

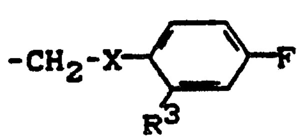

steht, worin
$R^2$ für Wasserstoff, Fluor oder Chlor steht,
$R^3$ für Wasserstoff, Fluor oder Chlor steht
und
X für Sauerstoff, Schwefel oder eine $CH_2$-Gruppe steht.

3. Verfahren zur Herstellung von Hydroxyethyl-azolyl-oximethern der Formel

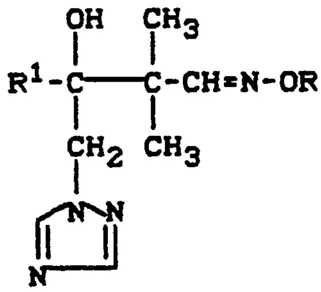

(I)

in welcher
R für Wasserstoff, Alkyl mit 1 bis 6 Kohlenstoffatomen oder Alkenyl mit 2 bis 6 Kohlenstoffatomen steht und
$R^1$ für die Reste der Formel

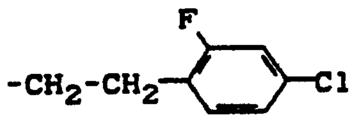

,

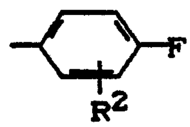

oder

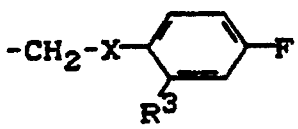

steht, worin
$R^2$ für Wasserstoff, Fluor oder Chlor steht,
$R^3$ für Wasserstoff, Fluor oder Chlor steht und
X für Sauerstoff, Schwefel oder eine $CH_2$-Gruppe steht,
sowie von deren Säureadditions-Salzen und Metallsalz-Komplexen, dadurch gekennzeichnet, daß man
a) Hydroxyethyl-azolyl-acetale der Formel

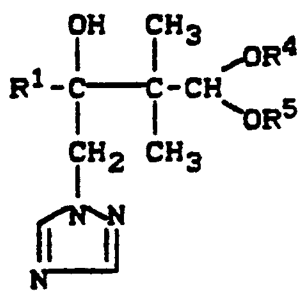

(IIa)

in welcher
$R^1$ die oben angegebene Bedeutung hat,
$R^4$ für Alkyl mit 1 bis 4 Kohlenstoffatomen steht und
$R^5$ für Alkyl mit 1 bis 4 Kohlenstoffatomen steht oder
$R^4$ und $R^5$ gemeinsam für eine $-CH_2-CH_2$-Gruppe stehen,
oder Hyroxyethyl-azolyl-aldehyde der Formel

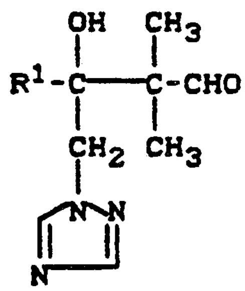

(IIb)

in welcher
$R^1$ die oben angegebene Bedeutung hat,
mit Hydroxylamin-Verbindungen der Formel

$H_2N\text{-}OR$ (III)

in welcher
R die oben angegebene Bedeutung hat,
oder mit Hydrochloriden von Hydroxylamin-Verbindungen der Formel (III) in Gegenwart eines Verdünnungsmittels und gegebenenfalls in Gegenwart eines Katalysators umsetzt,
oder
b) Hydroxyethyl-azolyl-oxim-Derivate der Formel

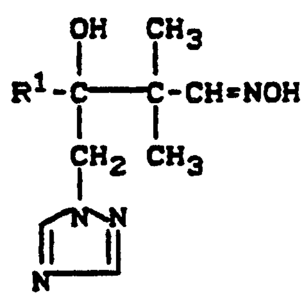

(Ia)

in welcher
$R^1$ die oben angegebene Bedeutun ghat,
mit Halogen-Verbindungen der Formel

$Hal\text{-}R^6$ (IV)

in welcher
Hal für Chlor, Brom oder Iod steht und
$R^6$ für Alkyl mit 1 bis 6 Kohlenstoffatomen oder Alkenyl mit 2 bis 6 Kohlenstoffatomen steht,
in Gegenwart eines Verdünnungsmittels und gegebenenfalls in Gegenwart eines Säurebindemittels umsetzt,
oder
c) Oxirane der Formel

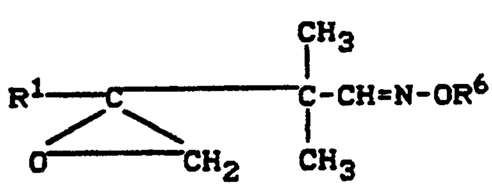

(V)

in welcher
$R^6$ und $R^1$ die oben angegebene Bedeutung haben,
mit 1,2,4-Triazol der Formel

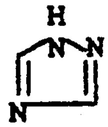

(VI)

in Gegenwart eines Verdünnungsmittels und gegebenenfalls in Gegenwart eines Säurebindemittels umsetzt, und gegebenenfalls anschließend an die so erhaltenen Verbindungen der Formel (I) eine Säure oder ein Metallsalz addiert.

4. Fungizide Mittel, gekennzeichnet durch einen Gehalt an mindestens einem Hydroxyethyl-azolyl-oximether der Formel (I) gemäß Anspruch 1 bzw. einem Säureadditions-Salz oder Metallsalz-komplex eines Hydroxyethyl-azolyl-oximethers der Formel (I).

5. Verwendung von Hydroxyethyl-azolyl-oximethern der Formel (I) gemäß Anspruch 1 bzw. von deren Säureadditionssalzen und Metallsalz-komplexen zur Bekämpfung von Pilzen.

6. Verfahren zur Bekämpfung von Pilzen, dadurch gekennzeichnet, daß man Hydroxyethyl-azolyl-oximether der Formel (I) gemäß Anspruch 1 bzw. deren Säueradditions-Salze oder Metallsalz-Komplexe auf die Pilze und/oder deren Lebensraum ausbringt.

7. Verfahren zur Herstellung von fungiziden Mitteln, dadurch gekennzeichnet, daß man Hydroxyethylazolyl-oximether der Formel (I) gemäß Anspruch 1 bzw. deren Säureadditions-Salze oder Metallsalzkomplexe mit Streckmitteln und/oder oberflächenaktiven Stoffen vermischt.

8. Oxirane der Formel

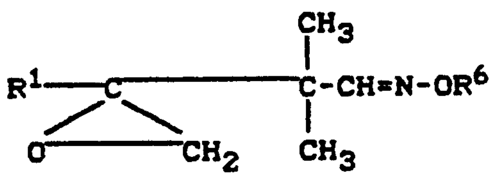

(V)

in welcher
$R^1$ für die Reste der Formel

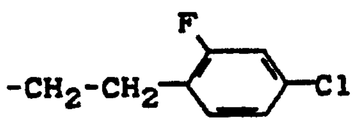

,

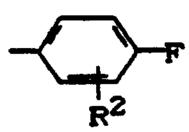

oder

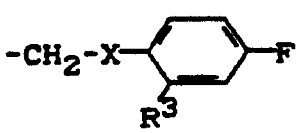

steht, worin
$R^2$ für Wasserstoff, Fluor oder Chlor steht,
$R^3$ für Wasserstoff, Fluor oder Chlor steht
und
$R^6$ für Alkyl mit 1 bis 6 Kohlenstoffatomen oder Alkenyl mit 2 bis 6 Kohlenstoffatomen steht.

9. Verfahren zur Herstellung von Oxiranen der Formel

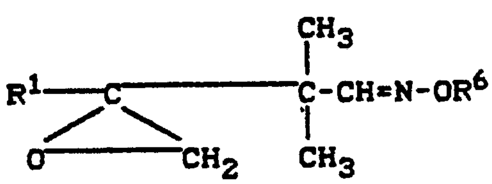

(V)

in welcher

$R^1$ für die Reste der Formel

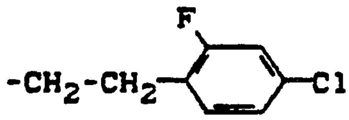 , 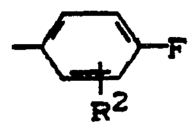 oder

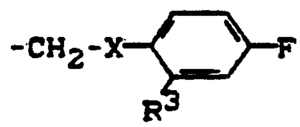

steht, worin
$R^2$ für Wasserstoff, Fluor oder Chlor steht,
$R^3$ für Wasserstoff, Fluor oder Chlor steht und
X für Sauerstoff, Schwefel oder eine $CH_2$-Gruppe steht,
und
$R^6$ für Alkyl mit 1 bis 6 Kohlenstoffatomen oder Alkenyl mit 2 bis 6 Kohlenstoffatomen steht,
dadurch gekennzeichnet, daß man Keto-oxim-Derivate der Formel

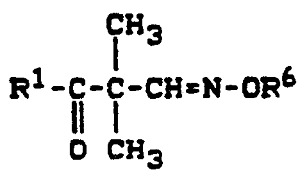 (XIV)

in welcher
$R^1$ und $R^6$ die oben angegebene Bedeutung haben,
α) mit Dimethyloxosulfonium-methylid der Formel

$$\overset{\delta+}{(CH_3)_2SO}\overset{\delta-}{CH_2} \qquad (IX)$$

in Gegenwart eines Verdünnungsmittels umsetzt,
oder
β) mit Trimethylsulfonium-methylsulfat der Formel

$[(CH_3)_3S^{\oplus}]\ CH_3SO_4^{\ominus}$ (X)

in Gegenwart eines inerten organischen Verdünnungsmittels und in Gegenwart einer Base umsetzt.

10. Hydroxyethyl-azolyl-oximether der Formel

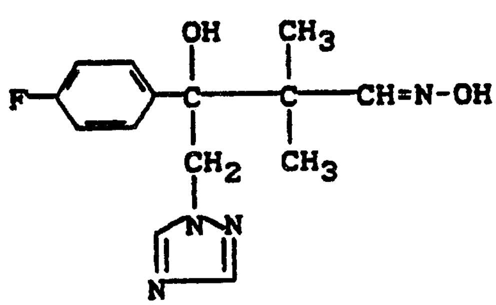

Europäisches Patentamt

# EUROPÄISCHER RECHERCHENBERICHT

Nummer der Anmeldung

EP 88111505.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP - A2 - 0 141 204 (BAYER)<br>* Formel I,V; Beispiele 4,22; Ansprüche 5,6,7 *<br>-- | 1,8,4,<br>5,6,10 | C 07 D 249/08<br>C 07 D 303/36<br>A 01 N 43/653 |
| A | EP - A2 - 0 176 998 (BAYER)<br>* Anspruch 1; Formel V *<br>---- | 1,8 | |
| | | | RECHERCHIERTE SACHGEBIETE (Int. Cl.4) |
| | | | C 07 D 249/00<br>C 07 D 303/00 |

Der vorliegende Recherchenbericht wurde für alle Patentansprüche erstellt.

| Recherchenort | Abschlußdatum der Recherche | Prüfer |
|---|---|---|
| WIEN | 20-09-1988 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82